Europäisches Patentamt

European Patent Office

Office européen des brevets

(19)

(11) Veröffentlichungsnummer : **0 168 723**
**B1**

(12) # EUROPÄISCHE PATENTSCHRIFT

(45) Veröffentlichungstag der Patentschrift :
07.03.90

(51) Int. Cl.⁵ : **C 07 H 15/04**

(21) Anmeldenummer : 85108316.2

(22) Anmeldetag : 05.07.85

(54) Verfahren zur Herstellung von Glykosiden aus Glykosylfluoriden.

(30) Priorität : 14.07.84 DE 3426074

(43) Veröffentlichungstag der Anmeldung :
22.01.86 Patentblatt 86/04

(45) Bekanntmachung des Hinweises auf die Patenter-
teilung : 07.03.90 Patentblatt 90/10

(84) Benannte Vertragsstaaten :
AT BE CH DE FR GB IT LI NL SE

(56) Entgegenhaltungen :
TETRAHEDRON LETTERS, Band 25, Nr. 13, 1984,
Seiten 1379-1382, Pergamon Press Ltd, GB; S. HASHI-
MOTO et al.: "Glycosylation using glucopyranosyl
fluorides and silicon-based catalysts. Solvent depen-
dency of the stereoselection"
JOURNAL OF THE AMERICAN CHEMICAL SOCIETY,
Band 106, Nr. 15, 25. Juli 1984, Seiten 4189-4192,
American Chemical Society; K.C. NICOLAOU et al.:
"Practical synthesis of oligosaccharides. Partial
synthesis of Avermectin B1a"
JOURNAL OF THE CHEMICAL SOCIETY-CHEMICAL
COMMUNICATIONS, Nr. 17, 1984, Seiten 1155-1156;
K.C. NICOLAOU et al.: "Reactions of glycosyl fluori-
des. Synthesis of O-, S-, and N-glycosides"
CARBOHYDRATE RESEARCH, Band 149, 1986, Seiten
347-361, Elsevier Science Publishers B.V., Amster-
dam, NL; M. KREUZER et al.: "Aufbau von Oligosac-
chariden mit Glykosylfluoriden unter Lewissäure-
Katalyse"

(73) Patentinhaber : HOECHST AKTIENGESELLSCHAFT
Postfach 80 03 20
D-6230 Frankfurt am Main 80 (DE)

(72) Erfinder : Thiem, Joachim, Prof. Dr.
Asbeckweg 31
D-4400 Münster (DE)
Erfinder : Deger, Hans-Matthias, Dr.
Am Rheingauer Weg 8
D-6238 Hofheim am Taunus (DE)
Erfinder : Fritsche-Lang, Wolfram, Dr.
Rhönstrasse 7
D-6140 Bensheim (DE)
Erfinder : Schlingmann, Merten, Dr.
Schneidhainer Strasse 32a
D-6240 Königstein/Taunus (DE)
Erfinder : Kreuzer, Matthias
Nottulner Landweg 21
D-4400 Münster (DE)

## Beschreibung

Es ist bekannt, daß Glukopyranosylfluoride mit Trimethylsilyläthern bzw. auch mit ungeschützten Alkoholen glykosidiert werden können, wenn man als Katalysatoren Siliciumtetrafluorid oder Trimethylsilyl-trifluor-methansulfonate (TMSOTF) einsetzt. Als Reaktionsmedium dient hierbei einerseits Acetonitril, das überwiegend zu den β-Glykosiden führt, andererseits Diäthyläther, der überwiegend zum α-Anomeren führt (S. Hashimoto, M. Hayashi u. R. Noyori, Tetrahedron Letters 25 (1984) 1379-1382).

Von den beiden verwendeten Katalysatoren ist TMSOTF zwar billiger und leichter zu handhaben, aber weniger wirksam als das Siliciumtetrafluorid, das ein stark korrosiv wirkendes und aggressives Gas ist. Die Aufarbeitung ist beim Einsatz beider Katalysatoren aufwendig, besonders beim TMSOTF, da hier die Bildung von Trifluormethansulfonsäure unbedingt vermieden werden muß.

Es wurde nun gefunden, daß sich anstelle des gasförmigen Siliciumtetrafluorids Fluoride von Metallen der 4. und 5. Gruppe des Periodensystems, die eine Ordnungszahl von mindestens 22 haben, in den höheren, stabilen und nicht oder nur schwach oxidierend wirkenden Oxidationsstufen besonders vorteilhaft für diese Glykosidierungsreaktion eignen. Von den Metallfluoriden, die auch Übergangsmetallfluoride umfassen, kommen beispielsweise Zinn- und Zirkontetrafluorid, Vanadin- und Antimonpentafluorid und insbesondere Titantetrafluorid in Betracht. Das ölige Vanadin- und Antimonpentafluorid ist im Reaktionssystem löslich und wirkt somit in homogener Phase, die übrigen genannten Fluoride sind fest und nicht oder nur wenig löslich, was ihre Handhabung und Abtrennung sehr erleichtert. Daher wird die Reaktion in heterogener Phase, insbesondere mit Titantetrafluorid, bevorzugt.

Die Reaktion wird im allgemeinen in einem für Reaktionen von Zuckern üblichen Lösungsmittel oder inerten Medium durchgeführt, wie Acetonitril, Diäthyläther, Methylenchlorid und/oder Nitrobenzol bzw. Gemischen davon mit Benzol, Toluol u. dgl. Im allgemeinen wendet man Temperaturen von −40 bis +60, vorzugsweise von −20 bis +30 °C an und arbeitet bei Atmosphärendruck, wenngleich man auch bei erhöhtem oder vermindertem Druck arbeiten kann.

Je nach Reaktivität und insbesondere sterischer Anordnung der Gruppen in den Reaktionsteilneh-mern werden die Fluoride in Mengen von 1 bis 200, bevorzugt 10 bis 120 und insbesondere bis 100, Mol-%, bezogen auf das Glykosylfluorid, eingesetzt.

Als Glykosylfluoride werden geschützte Hexopyranosylfluoride eingesetzt, vor allem solche, die sich von der Glukose, Galactose und Mannose ableiten. Einsetzbar sind aber auch Derivate dieser Zucker, wie 2-Desoxy- bzw. 2-Amino-2-desoxy-glukose, -galactose und -mannose sowie die Glukuron-, Galacturon- und Mannuronsäuren und ihre Derivate, insbesondere die Ester, Amide und Nitrile. Als Schutzgruppe kommt nicht nur die in dem bekannten Verfahren eingesetzte Benzyl-Schutz-gruppe in Betracht, sondern erfindungsgemäß auch Acyl-Schutzgruppen, vorzugsweise die Acetyl- und die Benzoylgruppe. Die Wahl der Schutzgruppe kann — abhängig vom erfindungsgemäß verwendeten Fluorid und einem etwa eingesetzten Lösungsmittel — einen Einfluß auf das Verhältnis der gebildeten Stereoisomeren haben.

Die Aglykone werden erfindungsgemäß unblockiert oder in Form ihrer Silyläther, z. B. der Triäthyl-, Tripropyl-, Triisopropyl- und Tributyläther, oder auch gemischter Äther wie dem t-Butyl-dimethylsilyläther, insbesondere aber als Trimethylsilyläther eingesetzt. Dabei kann die Reaktivität in Abhängigkeit von der Natur der Alkylgruppe schwanken. Als Aglykon kommen z. B. geradkettige und verzweigte aliphatische einwertige primäre, sekundäre und tertiäre $C_1$—$C_{10}$ und einwertige cycloaliphatische $C_5C_7$-Alkohole, vor allem aber Monosaccharide, aber auch Disaccharide in Betracht, insbesondere aus der Glukose-, Galactose- und Mannose-Reihe, deren nicht zu glykosidierende Hydroxygruppen durch geeignete Schutzgruppen blockiert sind. Als Schutzgruppen können hier je nach den Erfordernissen Acetale oder Ketale, Ätherfunktionen wie Benzyl- oder Triphenylmethyläther oder Acyl- bzw. Aryloxy- oder Alkoxycarbonyl-Gruppen zur Anwendung kommen. Besonders interessant als Schutzgruppen sind auch Epoxide (im Glykon oder Aglykon), welche unter den Bedingungen der erfindungsgemäßen Glykosid-Synthese stabil sind und in einem folgenden Reaktionsschritt leicht geöffnet werden können. Auch phenolische Aglykone sowie komplexe Steroidalkohole wie 5α-Cholestan-3β-ol lassen sich erfolgreich glykosidieren.

Die Verfahrensprodukte sind grundsätzlich bekannt und eignen sich als Zwischenprodukte für eine Vielzahl von Synthesen. Sie eignen sich auch als Modellsubstanzen für die Untersuchung der Wechselwirkung von Antigenen mit Partialstrukturen von Zellmembranen und sind aus diesem Grund als Immunmodulatoren von Interesse (K. M. Yamada u. K. Olden, Nature 275 (1978) 179-184, R. Neumeier, Biol. in unserer Zeit 13 (1983) 33-38).

Die Erfindung wird durch die nachstehenden Beispiele näher erläutert:

## Beispiele

1) 200 mg (0,37 mMol) 2,3,4,6-Tetra-O-benzyl-β-D-glucopyranosylfluorid und 123 mg (0,37 mMol) 1,2 ; 3,4-Di-O-isopropyliden-6-O-(trimethylsilyl)-α-D-galactopyranose wurden in 3 ml wasserfreiem Acetonitril gelöst und bei 0 °C mit 23 mg (0,19 mMol) Titan (IV) fluorid versetzt. Nach zweistündigem Rühren bei 0 °C, wobei der Verlauf der Reaktion dünnschichtchromatographisch mit Methylenchlorid/Diäthyläther als Laufmittel (20 : 1 v/v) kontrolliert wurde, wurde die Lösung eingeengt, der Rückstand in Chloroform aufgenommen und über Kieselgel (2 g) filtriert. Nach Abziehen des Lösungsmittels erhielt man ein Gemisch aus 6-O-(2,3,4,6-Tetra-O-benzyl-α- und β-D-glucopyranosyl)-1,2 ; 3,4-di-O-isopropyliden-α-D-galactopyranose : 255 mg (88 % Ausbeute).

Das Anomeren-Verhältnis betrug lt. $^1$H-NMR-Analyse α : β = 13 : 87. Die Anomeren wurden säulenchromatographisch getrennt (Kieselgel, Methylenchlorid/Diäthyläther 20 : 1 v/v).

α-Glukosid : $^1$H-NMR δ = 4,98 (1'-H, J(1', 2') = 3,8 Hz),
5,50 (1-H, J(1, 2) = 5,0 Hz).

$[\alpha]_D^{20}$ : + 11˚ (c = 0,87, CHCl$_3$).

β-Glukosid : $^1$H-NMR δ = 4,45 (1'-H, J(1', 2') = 7,8 Hz),
5,57 (1-H, J(1, 2) = 5,0 Hz).

$[\alpha]_D^{20}$ : − 32˚ (c = 0,87, CHCl$_3$).

2) 113 mg (0,21 mMol) 2,3,4,6-Tetra-O-benzyl-β-D-glucopyranosylfluorid und 36 mg (0,21 mMol) Trimethylsilylcyclohexyläther wurden in 2 ml wasserfreiem Acetonitril gelöst und bei 0 ˚C mit 27 mg (0,21 mMol) Titan(IV)fluorid versetzt. Nach 12 Stunden bei 0 ˚C, wobei der Verlauf der Reaktion dünnschichtchromatographisch mit Petroläther/Essigester (4 : 1 v/v) kontrolliert wurde, die Lösung eingeengt, der Rückstand in Methylenchlorid aufgenommen und über Kieselgel (2 g) filtriert. Nach Abziehen des Lösungsmittels erhielt man ein Gemisch aus Cyclohexyl-2,3,4,6-tetra-O-benzyl-α- und β-D-glucopyranosid : 99 mg (76 % Ausbeute). Das Anomeren-Verhältnis betrug nach Trennung α : β = 69 : 31.

Die Anomeren wurden säulenchromatographisch getrennt (Kieselgel, Methylenchlorid).

α-Glukosid : $^1$H-NMR δ = 4,94 (1-H, J(1, 2) = 3,8 Hz).

$[\alpha]_D^{20}$ : + 42,5˚ (c = 0,14, CHCl$_3$).

β-Glukosid : $^1$H-NMR δ = 4,49 (1-H, J(1, 2) = 7,8 Hz).

Schmp. 104-105 ˚C

$[\alpha]_D^{20}$ : + 9˚ (c = 0,73, CHCl$_3$).

3A) 36,4 mg (0,067 mMol) 2,3,4,6-Tetra-O-benzyl-β-D-glucopyranosylfluorid und 14,5 mg (0,067 mMol) 1,6 ; 2,3-Dianhydro-4-O-trimethylsilyl-β-D-mannopyranose wurden in 1 ml wasserfreiem Acetonitril gelöst und bei 0 ˚C mit 8,3 mg (0,067 mMol) Titan(IV)fluorid versetzt. Nach 2 Stunden bei 22 ˚C wurde die Lösung filtriert und eingeengt, der Rückstand in Methylenchlorid aufgenommen und über Kieselgel (2 g) filtriert. Nach Abziehen des Lösungsmittels unter vermindertem Druck wurde der Rückstand säulenchromatographisch (Kieselgel, Methylenchlorid/Diäthyläther 20 : 1 v/v) gereinigt. Man erhielt ein Gemisch aus 1,6 ; 2,3-Dianhydro-4-O-(2,3,4,6-tetra-O-benzyl-α- und -β-D-glucopyranosyl)-β-D-manno-pyranose : 45 mg (44 % Ausbeute). Das Anomeren-Verhältnis betrug lt. $^1$H-NMR-Analyse α : β = 45 : 55.

3B) 1,1 g (2,03 mMol) 2,3,4,6-Tetra-O-benzyl-β-D-glucopyranosylfluorid und 293 mg (2,03 mMol) 1,6 ; 2,3-Dianhydro-β-D-mannopyranose wurden in 10 ml wasserfreiem Acetonitril gelöst und bei 0 ˚C mit 126 mg (1,02 mMol) Titan(IV)fluorid versetzt. Nach 2 Stunden bei 0 ˚C wurde die Lösung über 10 g Kieselgel filtriert, das Kieselgel mit Essigsäureäthylester nachgespült und die vereinigten Lösungen eingeengt. Man erhielt 1,24 g (92 %) eines Rohproduktes, das neben Spuren der Ausgangsverbindung ein Anomerengemisch im Verhältnis von α : β = 45 : 55 enthielt. Die Trennung der Anomeren erfolgte säulenchromatographisch auf Kieselgel mit Methylenchlorid/Diäthyläther 20 : 1.

Ausbeute : α-Glycosid : 460 mg (34 %)
β-Glykosid : 540 mg (40 %)

Die nach den Beispielen 3A) und 3B) hergestellten Glykoside haben folgende Kenndaten :

α-Glukosid : $^1$H-NMR : δ = 5,62 (1-H, J(1, 2) = 3,1 Hz),
4,87 (1'-H, J(1', 2') = 3,6 Hz).

Schmp. : 131-135 ˚C

$[\alpha]_D^{20}$ : + 32,5˚ (c = 0,63, CHCl$_3$).

β-Glykosid : $^1$H-NMR : δ = 5,74 (1-H, J(1, 2) = 2,6 Hz),
4,56 (1'-H, J(1', 2') = 7,8 Hz).

Schmp. : 143-144 ˚C

$[\alpha]_D^{20}$ : − 2,3˚ (c = 0,985, CHCl$_3$).

4) 80 mg (0,23 mMol) 2,3,4,6-Tetra-O-acetyl-β-D-glucopyranosylfluorid und 79 mg (0,23 mMol) 1,2 ; 3,4-Di-O-isopropyliden-6-O-(trimethylsilyl)-α-D-galactopyranose wurden in 2 ml wasserfreiem Acetonitril mit 16 mg (0,13 mMol) Titan(IV)fluorid bei Raumtemperatur gerührt. Nach 2 Stunden waren die Ausgangsverbindungen nur noch in Spuren dünnschichtchromatographisch nachweisbar (Methylenchlorid/Essigester 4 : 1 v/v). Die Lösung wurde eingeengt, der Rückstand in Methylenchlorid aufgenommen und über Kieselgel filtriert. Es wurde lt. $^1$H-NMR-Analyse ausschließlich 6-O-(2,3,4,6-Tetra-O-acetyl-β-D-glucopyranosyl)-1,2 ; 3,4-di-O-isopropyliden-α-D-galactopyranose erhalten, welche säulenchromatographisch (Kieselgel, Methylenchlorid/Essigester 4 : 1 v/v) oder durch Umkristallisieren aus Diäthyläther/Petroläther gereinigt wurde. Ausbeute : 89 mg (66 %).

$$\beta\text{-Glukosid}: {}^1\text{H-NMR}: \delta = 4,61 \; (1'\text{-H}, J(1', 2') = 7,9 \, \text{Hz}),$$
$$5,48 \; (1\text{-H}, J(1, 2) = 4,8 \, \text{Hz}).$$

$$\text{Schmp.} : 140\text{-}142 \, ^\circ\text{C}$$

$$[\alpha]_D^{20} : -53^\circ \; (c = 1,05, \text{CHCl}_3).$$

5)-7) Die in den Beispielen 1, 3A und 4 beschriebenen Reaktionen wurden in Gegenwart von gleichen Molmengen Zinntetrafluorid anstelle von Titantetrafluorid als Katalysator bei 22 °C mit Acetonitril als Lösungsmittel durchgeführt.

| Nr. | t(h) | Ausbeute (%) | α:β-Verhältnis |
|---|---|---|---|
| 5 | 2 | 89 | 15:85 |
| 6 | 72 | 49 | 31:69 |
| 7 | 24 | 44 | 0:100 |

8) 600 mg (1,78 mMol) Methyl (2,3,4-tri-O-acetyl-β-D-galactopyranosylfluorid)uronat und 490 mg (1,77 mMol) 1,6-Anhydro-2-azido-4-O(-benzyl-β-D-glucopyranose wurden in 10 ml wasserfreiem Acetonitril gelöst und bei Raumtemperatur mit 100 mg (0,81 mMol) Titan(IV)fluorid und 1 g gepulvertem Molekularsieb 3Å versetzt. Nach zweistündigem Rühren wurde die Lösung über 5 g Kieselgel filtriert, dann eingeengt und das Rohprodukt (1,06 g) chromatographisch (Kieselgel, n-Hexan/Essigester, 2 : 1 v/v) gereinigt. Ausbeute an reiner 3-O-[Methyl(2,3,4-tri-O-acetyl-β-D-galactopyranosyl)uronat]-1,6-anhydro-2-azido-4-O-benzyl-β-D-glucopyranose : 710 mg (67 %).

$${}^1\text{H-NMR} \; (\text{CDCl}_3): \delta = 5,19 \; (1\text{-H}), 4,49 \; (1'\text{-H}, J(1', 2') = 7,9 \, \text{Hz}).$$

$$\text{Schmp.} : 68 \, ^\circ\text{C}$$

$$[\alpha]_D^{20} = 49,6^\circ \; (c = 1,97, \text{CDCl}_3).$$

## Patentansprüche

1. Verfahren zur Herstellung von Glykosiden aus geschützten Hexopyranosylfluoriden und Aglykonen bzw. deren Silyläthern in Gegenwart von Fluoriden, dadurch gekennzeichnet, daß die Reaktion in Gegenwart eines Metallfluorids der 4. oder 5. Gruppe des Periodensystems der Elemente mit einer Ordnungszahl von mindestens 22 in einer höheren, stabilen und nicht oder nur schwach oxidierend wirkenden Oxidationsstufe eingesetzt werden.

2. Verfahren nach Anspruch 1, dadurch gekennzeichnet, daß als Fluorid Titantetrafluorid, Zirkontetrafluorid, Zinntetrafluorid, Vanadinpentafluorid oder Antimonpentafluorid eingesetzt werden.

3. Verfahren nach Anspruch 1 oder 2, dadurch gekennzeichnet, daß die Reaktion in heterogener Phase mit einem festen, im Reaktionssystem nicht oder nur wenig löslichen Fluorid durchgeführt wird.

4. Verfahren nach einem oder mehreren der vorhergehenden Ansprüche, dadurch gekennzeichnet, daß das Fluorid in einer Menge von 1 bis 200 Mol.-% eingesetzt wird, bezogen auf das Hexopyranosylfluorid.

5. Verfahren nach Anspruch 4, dadurch gekennzeichnet, daß die Menge 10 bis 120 und insbesondere bis 100 Mol.-% beträgt.

6. Verfahren nach einem oder mehreren der vorhergehenden Ansprüche, dadurch gekennzeichnet, daß die Reaktion in Gegenwart eines Lösungsmittels oder inerten Mediums durchgeführt wird.

7. Verfahren nach einem oder mehreren der vorhergehenden Ansprüche, dadurch gekennzeichnet, daß als Silyläther der Trimethylsilyläther verwendet wird.

8. Verfahren nach einem oder mehreren der vorhergehenden Ansprüche, dadurch gekennzeichnet, daß die Reaktion bei Temperaturen von − 40 bis + 60, vorzugsweise von − 20 bis + 30 °C durchgeführt wird.

9. Verfahren nach einem oder mehreren der vorhergehenden Ansprüche, dadurch gekennzeichnet, daß das Aglykon ein einwertiger aliphatischer Alkohol mit 1 bis 10 C-Atomen oder ein einwertiger cycloaliphatischer Alkohol mit 5 bis 7 C-Atomen oder ein Saccharid, insbesondere aber ein Monosaccharid ist.

## Claims

1. A process for the preparation of glucosides from protected hexopyranosyl fluorides and aglycones or the silyl ethers of the latter in the presence of fluorides, wherein the reaction is carried out in the presence of a metal fluoride of the 4th or 5th group of the Periodic Table of the Elements, having an atomic number of at least 22, in a higher, stable and non-oxidizing or only slightly oxidizing oxidation state.

2. The process as claimed in claim 1, wherein titanium tetrafluoride, zirconium tetrafluoride, tin tetrafluoride, vanadium pentafluoride or antimony pentafluoride is used as the fluoride.

3. The process as claimed in claim 1 or 2, wherein the reaction is carried out in a heterogeneous phase with a solid fluoride which is insoluble or only slightly soluble in the reaction system.

4. The process as claimed in one or more of the preceding claims, wherein the fluoride is employed in an amount from 1 to 200 mol%, relative to the hexopyranosyl fluoride.

5. The process as claimed in claim 4, wherein the amount is 10 to 120 and especially up to 100 mol%.

6. The process as claimed in one or more of the preceding claims, wherein the reaction is carried out in the presence of a solvent or inert medium.

7. The process as claimed in one or more of the preceding claims, wherein the trimethylsilyl ether is used as the silyl ether.

8. The process as claimed in one or more of the preceding claims, wherein the reaction is carried out at temperatures from − 40 to + 60, preferably from − 20 to + 30 ˚C.

9. The process as claimed in one or more of the preceding claims, wherein the aglycone is a monohydric aliphatic alcohol having 1 to 10 carbon atoms or a monohydric cycloaliphatic alcohol having 5 to 7 carbon atoms or a saccharide, but especially a monosaccharide.

## Revendications

1. Procédé pour préparer des glycosides à partir de fluorures d'hexopyrannosyles protégés et d'aglycones ou de leurs éthers silyliques, en présence de fluorures, procédé caractérisé en ce qu'on effectue la réaction en présence d'un fluorure d'un métal qui appartient au groupe 4 ou au groupe 5 de la classification périodique des éléments et qui a un numéro atomique d'au moins 22, à un stade d'oxydation stable, assez élevé et n'ayant pas une action oxydante ou n'en ayant qu'une faible.

2. Procédé selon la revendication 1, caractérisé en ce qu'on utilise, comme fluorure, le tétrafluorure de titane, le tétrafluorure de zirconium, le tétrafluorure d'étain, le pentafluorure de vanadium ou le pentafluorure d'antimoine.

3. Procédé selon l'une des revendications 1 et 2, caractérisé en ce qu'on effectue la réaction en phase hétérogène avec un fluorure solide· qui n'est pas soluble ou qui n'est que peu soluble dans le système réactionnel.

4. Procédé selon l'une quelconque des revendications précédentes, caractérisé en ce que le fluorure est mis en jeu en une quantité de 1 à 200 % en moles par rapport au fluorure d'hexopyrannosyle.

5. Procédé selon la revendication 4, caractérisé en ce que la quantité est de 10 à 120 et, plus particulièrement, à 100 % en moles.

6. Procédé selon l'une quelconque des revendications précédentes, caractérisé en ce qu'on effectue la réaction en présence d'un solvant ou d'un milieu inerte.

7. Procédé selon l'une quelconque des revendications précédentes, caractérisé en ce qu'on utilise, comme éther silylique, l'éther triméthylsilylique.

8. Procédé selon l'une quelconque des revendications précédentes, caractérisé en ce qu'on effectue la réaction à des températures de − 40 à + 60 ˚C, de préférence de − 20 à + 30 ˚C.

9. Procédé selon l'une quelconque des revendications précédentes, caractérisé en ce que l'aglycone est un mono-alcool aliphatique contenant de 1 à 10 atomes de carbone ou un mono-alcool cycloaliphatique contenant de 5 à 7 atomes de carbone ou un saccharide, mais plus particulièrement un monosaccharide.